# EUROPEAN PATENT APPLICATION

(11) **EP 4 420 607 A1**
(43) Date of publication of application: **28.08.2024**
(21) Application number: 23845351.8
(22) Date of filing: 14.07.2023
(51) Int. Cl.: A61B 5/0225, A44C 5/14

(54) **WEARABLE DEVICE AND WEARABLE SYSTEM**

(30) Priority: 29.07.2022 CN 202210908425
(71) Applicant: Huawei Technologies Co., Ltd., Shenzhen, Guangdong 518129 (CN)
(72) Inventor: XIE, Yun, Shenzhen, Guangdong 518129 (CN); YUAN, Shenglan, Shenzhen, Guangdong 518129 (CN); ZHAO, Menglong, Shenzhen, Guangdong 518129 (CN); GONG, Lexing, Shenzhen, Guangdong 518129 (CN); CHENG, Tianyu, Shenzhen, Guangdong 518129 (CN); WEI, Qichong, Shenzhen, Guangdong 518129 (CN); TANG, Chien Wei, Shenzhen, Guangdong 518129 (CN); YANG, Junjie, Shenzhen, Guangdong 518129 (CN)
(74) Representative: Körber, Martin Hans
(86) International application number: PCT/CN2023/107578
(87) International publication number: WO 2024/022139

(57) **Abstract**

Embodiments of this application provide a wearable device and a wearable system. The wearable device includes a device body, and a vent is provided on a side wall of the device body. One watch strap assembly includes a first watch strap body and an airbag, the airbag is detachably attached to an inner side of the first watch strap body, one end of the airbag includes a first air nozzle, the first air nozzle is in communication with one end of an air flow channel at one end of the first watch strap body, the other end of the air flow channel is in communication with the vent when the first watch strap body is connected to the side wall of the device body, an end that is of the first watch strap body and that is connected to the device body covers the vent, a pressure measurement assembly in the device body is in communication with the vent, and the pressure measurement assembly inflates the airbag through the vent. According to the wearable device provided in embodiments of this application, a good attaching effect between the airbag and a wrist in a wearing process and a blood pressure measurement process of the wearable device is implemented. This improves wearing comfort of the wearable device in the wearing process and the blood pressure measurement process, and avoids a problem that dust tends to gather at the vent in the wearable device.

## Description

This application claims priority to Chinese Patent Application No. 202210908425.7, filed with the China National Intellectual Property Administration on July 29, 2022 and entitled "WEARABLE DEVICE AND WEARABLE SYSTEM", which is incorporated herein by reference in its entirety.

### TECHNICAL FIELD

This application relates to the field of electronic device technologies, and in particular, to a wearable device and a wearable system.

### BACKGROUND

With development of science and technology, wearable devices are increasingly popular for users due to portability and intelligence of the wearable devices. In addition, users have increasing requirements for functions, appearance, use experience, and the like of the wearable devices. Wearable products have many functions such as being wearable close to the skin and detecting a physical health status at any time. In recent years, an increasing quantity of people begin to pay attention to accepting and using wearable products, such as wearable watches and wearable bands.

In the conventional technology, an airbag used to monitor health indicators such as blood pressure is disposed on a wearable device. When the airbag is disposed, a vent is usually provided on a bottom housing of the wearable device, the airbag is fastened to the bottom housing, and the airbag is in communication with the vent. After a watch strap is worn tightly on a wrist, the airbag may touch the wrist, to detect health indicators of a wearer.

However, in a wearing process of the wearable device, because a position at which the airbag is connected to the watch strap tends to cause uncomfortable wearing after the airbag is inflated, the vent is provided on the bottom housing of the wearable device, and consequently dust tends to gather at the vent.

### SUMMARY

Embodiments of this application provide a wearable device and a wearable system, to avoid a problem that dust tends to gather at a vent in a wearable device, implement a good attaching effect between an airbag and a wrist in a wearing process and a blood pressure measurement process of the wearable device, and therefore improve wearing comfort of the wearable device in the wearing process and the blood pressure measurement process.

An aspect of embodiments of this application provides a wearable device. The wearable device includes: a device body, where the device body includes a side wall, and a vent is provided on the side wall; at least one watch strap assembly, where at least one end of each watch strap assembly is connected to the side wall of the device body, one of the at least one watch strap assembly includes a first watch strap body and an airbag, the airbag is detachably attached to an inner side of the first watch strap body, one end of the airbag includes a first air nozzle, an air flow channel is provided at one end of the first watch strap body, the first air nozzle is in communication with one end of the air flow channel, the other end of the air flow channel is in communication with the vent when the first watch strap body is connected to the side wall of the device body, and an end that is of the first watch strap body and that is connected to the device body covers the vent; and a pressure measurement assembly, where the pressure measurement assembly is disposed in the device body, and the pressure measurement assembly is in communication with the vent, to enable the pressure measurement assembly to inflate the airbag through the vent.

In embodiments of this application, the device body, the at least one watch strap assembly, and the pressure measurement assembly are included. The vent is provided on the side wall of the device body, one of the at least one watch strap assembly includes the first watch strap body and the airbag, the airbag is detachably attached to the inner side of the first watch strap body, and one end of the airbag is provided with the first air nozzle, the first air nozzle is in communication with one end of the air flow channel that is of the first watch strap body and that is close to the device body, the other end of the air flow channel is in communication with the vent when the first watch strap body is connected to the side wall of the device body, and the end that is of the first watch strap body and that is connected to the device body covers the vent. In this way, when the wearable device is worn on a wrist, because the airbag is attached to the inner side of the first watch strap body, the first air nozzle of the airbag is connected to the air flow channel at one end of the first watch strap body, and the airbag and the first watch strap body are assembled into an overall structure and connected to the device body, the airbag can implement a good attaching function on the wrist after being inflated, so that wearing the airbag on the wrist is not uncomfortable in a wearing process and during blood pressure measurement of the wearable device. In this way, wear comfort of the wearable device in a wearing process and during blood pressure measurement is improved, and a problem of uncomfortable wearing of the wearable device is avoided. In addition, when one end that is of the first watch strap body and that is provided with the air flow channel is connected to the device body, the end that is of the first watch strap body and that is connected to the side wall of the device body covers the vent on the side wall of the device body, so that the vent of the device body is not exposed, and a problem that dust tends to gather at the vent of the wearable device is avoided. In addition, the airbag is located on the inner side of the first watch strap body, and does not occupy bottom housing space of the device body, so that the device body of the wearable device is thinner when the wearable device has a blood pressure measurement function. Therefore, the wearable device provided in embodiments of this application resolves problems that the wearable device is uncomfortable to wear during blood pressure measurement and dust tends to gather at a vent.

In a possible implementation, there are two watch strap assemblies, the two watch strap assemblies are a first watch strap assembly and a second watch strap assembly, and the first watch strap assembly includes the first watch strap body and the airbag; and the second watch strap assembly includes a second watch strap body and a watch buckle, one end of the second watch strap body is connected to the device body, the other end of the second watch strap body is connected to the watch buckle, and the watch buckle is configured to be detachably connected to one end of the first watch strap body.

In a possible implementation, one end of the first watch strap body has a first connection structure, the air flow channel is provided in the first connection structure, the first air nozzle is connected to one end of the first connection structure, and the other end of the first connection structure is detachably connected to a side wall of the device body.

In a possible implementation, one end that is of the first connection structure and that is connected to the device body has a second air nozzle in communication with the air flow channel, and the second air nozzle is sealed and connected to the vent.

In a possible implementation, the wearable device further includes: a first control button. The first control button is disposed on the device body, and the first control button is configured to control the first connection structure to be connected to or detached from the device body.

In a possible implementation, the first control button includes a button body and an elastic part, and the button body includes a pressing part and two fastening parts respectively located on two sides of the pressing part; the fastening part is configured to fit a fastening slot provided on the first connection structure, to connect the first connection structure to the device body; the pressing part is configured to move in a thickness direction of the device body when being pressed, to enable the fastening part to be detached from the fastening slot; and one end of the elastic part is connected to the pressing part, the other end of the elastic part is connected to the device body, and the elastic part is configured to enable the button body to extend or retract on the device body.

In a possible implementation, a plurality of first adjustment holes spaced apart are provided on the first watch strap body, and the first adjustment holes are configured to fasten the watch buckle.

In a possible implementation, a buckle nail is disposed on an outer side surface of the first watch strap body, a plurality of second adjustment holes spaced apart are disposed on the second watch strap body, and the buckle nail is configured to be fastened and connected to the second adjustment hole.

In a possible implementation, a loop that is capable of being passed through by the watch buckle is disposed at one end that is of the first watch strap body and that is disposed back to back the device body, and the loop is rotatably connected to the first watch strap body.

In a possible implementation, the watch buckle includes a watch buckle cover, a fastening post and a movable sliding buckle are disposed on the watch buckle cover, the fastening post is configured to fit one of the first adjustment holes, and the sliding buckle is configured to be fastened to another one of the first adjustment holes.

In a possible implementation, the watch buckle includes a base and an upper cover, where the upper cover is rotatably connected to the base, a gap that is capable of being passed through by one end of the first watch strap body is provided between the upper cover and the base; and a buckle pin for fastening to the first adjustment hole is disposed on a surface that is of the upper cover and that faces the base.

In a possible implementation, a detection electrode is further disposed on the watch buckle, a flexible circuit board is disposed in the second watch strap body, and the detection electrode is electrically connected to a circuit board in the device body through the flexible circuit board.

In a possible implementation, the wearable device further includes: a second connection structure and a second control button. One end of the second connection structure is connected to the second watch strap body, the other end of the second connection structure is connected to the device body, a conductive contact is disposed on the second connection structure, the conductive contact is electrically connected to the flexible circuit board, and a conductive terminal in electrical contact with the conductive contact is disposed on a surface that is of the device body and that is connected to the second connection structure; and
the second control button is disposed on the device body, and the second control button is configured to control the second connection structure to be connected to or detached from the device body.

In a possible implementation, both the first watch strap body and the second watch strap body are both of a flexible plastic material.

In a possible implementation, a tensile interlayer is disposed in the first watch strap body and the second watch strap body, or is disposed on inner sides of the first watch strap body and the second watch strap body.

In a possible implementation, a fastening press strip is disposed on one of the first watch strap body and the airbag, and a slot that fits the fastening press strip is provided on the other one of the watch strap body and the airbag.

In a possible implementation, a length of the first watch strap body is greater than a length of the second watch strap body, and the airbag extends along one end of the first watch strap body to the other end of the first watch strap body.

In a possible implementation, there is one watch strap assembly, a first end of a first watch strap body of the watch strap assembly is connected to the side wall of the device body, a second end of the first watch strap body is in sliding cooperation with the device body, and the second end of the first watch strap body is detachably connected to the first watch strap body.

In a possible implementation, the first watch strap body is a non-elastic woven strap.

In a possible implementation, one end of the first watch strap body includes a third connection structure, the third connection structure includes a first lug and a first end link, the air flow channel is provided in the first end link, and one end of the first end link is connected to the first end of the first watch strap body, and the other end of the first end link is connected to the side wall of the device body; and
the first lug is disposed to cover the first end link.

In a possible implementation, the third connection structure further includes a first buffer body, the first buffer body is disposed on the first end link, and the first buffer body is located between the first lug and the side wall of the device body.

In a possible implementation, the third connection structure further includes a cover plate and a fastener, the cover plate is pressed on the first air nozzle of the airbag, and the fastener is configured to fasten the cover plate to the first end link, to enable the airbag to be fastened to the first end link.

In a possible implementation, the wearable device further includes a fourth connection structure. The fourth connection structure includes a second lug, a second end link, and a spring bar, the second end link is connected to the second lug, and the second end link is connected to the device body; and
two ends of the spring bar are connected to the second lug, sliding space for being passed through by the second end of the first watch strap body is provided between the spring bar and the second lug, and the second end of the first watch strap body is slidably disposed around the spring bar.

In a possible implementation, a bonding component is disposed on the second end of the first watch strap body, and a connecting component that is bonded to the bonding component is disposed on a side that is of the first watch strap body and that is disposed back to back the airbag.

In a possible implementation, the fourth connection structure further includes a second buffer body, the second buffer body is disposed on the second lug, and the second buffer body is located between the second lug and the side wall of the device body.

In a possible implementation, the airbag is connected to the first watch strap body by using a hook and loop fastener.

In a possible implementation, the pressure measurement assembly includes a pressure difference sensor, an air path, and an air pump, and the vent is separately in communication with the pressure difference sensor and the air pump through the air path; and
a control valve is disposed in the air path.

Another aspect of embodiments of this application provides a wearable system. The wearable system includes a terminal device and the wearable device according to the first aspect, and the terminal device is communicatively connected to the wearable device.

### BRIEF DESCRIPTION OF DRAWINGS

FIG. 1 is a schematic three-dimensional diagram in a wearable device according to an embodiment of this application;
FIG. 2 is a schematic diagram of a structure in which a device body and a watch strap assembly in a wearable device are split according to an embodiment of this application;
FIG. 3 is a schematic diagram of a structure of one watch strap assembly in a wearable device according to an embodiment of this application;
FIG. 4A is a schematic diagram of a cross-sectional structure of a part of a device body and a watch strap assembly in a wearable device according to an embodiment of this application;
FIG. 4B is a schematic three-dimensional diagram of a wearable device in another direction according to an embodiment of this application;
FIG. 5A is a schematic diagram of a structure in which one watch strap assembly in a wearable device is split according to an embodiment of this application;
FIG. 5B is a schematic diagram of a structure of an airbag in a wearable device according to an embodiment of this application;
FIG. 5C is a schematic diagram of assembling a watch strap body and an airbag in a wearable device according to an embodiment of this application;
FIG. 5D is another schematic diagram of assembling a watch strap body and an airbag in a wearable device according to an embodiment of this application;
FIG. 6 is a schematic diagram of a structure of a first connection structure in a wearable device according to an embodiment of this application;
FIG. 7 is a schematic diagram of a structure of a part of a watch strap assembly in a wearable device according to an embodiment of this application;
FIG. 8A is a schematic diagram of a structure in which a watch strap assembly in a wearable device is split according to an embodiment of this application;
FIG. 8B is a schematic diagram of a device body in a wearable device according to an embodiment of this application;
FIG. 8C is a schematic diagram in which a device body and a first control button in a wearable device are split according to an embodiment of this application;
FIG. 9 is a schematic diagram of cooperation between a first connection structure and a first control button, and between a second control button, a second connection structure, and a bottom housing of a device body in a wearable device according to an embodiment of this application;
FIG. 10 is a schematic diagram of a structure of two watch strap assemblies in a wearable device according to an embodiment of this application;
FIG. 11 is a schematic diagram of cooperation between two watch strap assemblies of a wearable device when the watch strap assemblies are connected to each other according to an embodiment of this application;
FIG. 12 is a schematic diagram of cooperation between two watch strap assemblies of a wearable device when the watch strap assemblies are connected to each other according to an embodiment of this application;
FIG. 13 is a partial schematic diagram of two watch strap assemblies in a wearable device according to an embodiment of this application;
FIG. 14 is a schematic diagram in which a watch buckle of a second watch strap assembly in a wearable device is split according to an embodiment of this application;
FIG. 15A is a schematic cross-sectional diagram of two watch strap assemblies of a wearable device at a watch buckle according to an embodiment of this application;
FIG. 15B is a schematic diagram of a structure of a watch strap body and a tensile interlayer in a wearable device according to an embodiment of this application;
FIG. 15C is another schematic diagram of a structure of a watch strap body and a tensile interlayer in a wearable device according to an embodiment of this application;
FIG. 16 is another schematic three-dimensional diagram in a wearable device according to an embodiment of this application;
FIG. 17 is a schematic diagram of a detection electrode and a device body in a wearable device according to an embodiment of this application;
FIG. 18 is another schematic diagram of a structure of a second watch strap assembly in a wearable device according to an embodiment of this application;
FIG. 19 is another schematic diagram of a structure in which a second watch strap assembly in a wearable device is split according to an embodiment of this application;
FIG. 20 is a schematic diagram in which a watch buckle of a second watch strap assembly in a wearable device is split according to an embodiment of this application;
FIG. 21 is a schematic diagram in which a part of a watch buckle of the second watch strap assembly in a wearable device is split according to an embodiment of this application;
FIG. 22 is a schematic diagram of a structure of cooperation between two watch strap assemblies in a wearable device according to an embodiment of this application;
FIG. 23 is a schematic diagram of a cross-sectional structure of two watch strap assemblies in a wearable device obtained when the watch strap assemblies cooperate according to an embodiment of this application;
FIG. 24 is a schematic diagram of a structure in which a device body and a watch strap assembly in a wearable device are split according to an embodiment of this application;
FIG. 25 is a schematic diagram of a structure in which a watch strap assembly in a wearable device is split according to an embodiment of this application;
FIG. 26 is a schematic diagram of a partial sectional view of a watch strap assembly and a device body in a wearable device according to an embodiment of this application;
FIG. 27 is a schematic diagram of assembling a first end of a watch strap assembly and a first end link in a third connection structure in a wearable device according to an embodiment of this application;
FIG. 28 is a schematic diagram of assembling a first end link and a first buffer body in a third connection structure, and a first end of a watch strap assembly in a wearable device according to an embodiment of this application;
FIG. 29 is a schematic diagram of assembling a first end link, a first buffer body, and a first lug in a third connection structure, and a first end of a watch strap assembly in a wearable device according to an embodiment of this application;
FIG. 30 is a schematic diagram of assembling a first end link, a first buffer body, and a first lug in a third connection structure and an airbag, and a first end of a watch strap assembly in a wearable device according to an embodiment of this application;
FIG. 31 is a schematic diagram of assembling a first end link, a first buffer body, a first lug, and a cover plate in a third connection structure and an airbag, and a first end of a watch strap assembly in a wearable device according to an embodiment of this application;
FIG. 32 is a schematic diagram of assembling a second end link, a second buffer body, and a second lug in a fourth connection mechanism in a wearable device according to an embodiment of this application; and
FIG. 33 is a schematic diagram of assembling a second end link, a second buffer body, a spring bar, and a second lug in a fourth connection mechanism in a wearable device according to an embodiment of this application.

Descriptions of reference numerals:
100: wearable device;
110: device body; 111: side wall; 112: bottom wall; 1111: vent; 113: conductive terminal; 114: bottom housing;
120: watch strap assembly; 120a: first watch strap assembly; 120b: second watch strap assembly; 121: first watch strap body; 1211: first adjustment hole; 1212: buckle nail; 1213: loop; 1214: slot; 121a: first end; 121b: second end;
122: second watch strap body; 1221: watch buckle; 1221a: watch buckle cover; 1221b: sliding buckle; 1221c: fastening post; 1221h: base; 1221i: upper cover; 1221g: buckle pin; 1222: second adjustment hole; 123: airbag; 1231: first air nozzle; 1233: fastening press strip; 124: detection electrode; 125: flexible circuit board; 1251: adhesive layer; 126: tensile interlayer;
130: pressure measurement assembly; 131: pressure difference sensor; 132: air path; 133: control valve; 134: air pump;
141: first connection structure; 1411: air flow channel; 1412: second air nozzle; 142: second connection structure; 1421: conductive contact; 143: third connection structure; 1431: first lug; 1432: first buffer body; 1433: first end link; 1434: cover plate; 1435: fastener; 144: fourth connection structure; 1441: second buffer body; 1442: second lug; 1443: second end link; 1444: spring bar; 141a and 142a: fastening slots;
150: first control button; 151: button body; 1511: pressing part; 1512: fastening part; 152: elastic part; and
160: second control button.

### DESCRIPTION OF EMBODIMENTS

An embodiment of this application provides a wearable device 100. The wearable device 100 may be an intelligent wearable device worn on a wrist. Specifically, the intelligent wearable device worn on the wrist may be a wearable device like a smart watch, a sports watch, or a smart band. This is not limited in this embodiment. The smart watch is used as an example for description in this embodiment.

The wearable device 100 may obtain body sign data of a wearer by integrating a sensor. In this embodiment of this application, the wearable device 100 has a blood pressure measurement function.

In the conventional technology, in a wearable device having a blood pressure measurement, the wearable device usually includes a device body, a watch strap, and an airbag. A vent is provided on a bottom housing of the device body, the airbag is fastened to the bottom housing of the device body and is in communication with the vent, and two ends of the airbag are detachably connected to the watch strap. However, because the vent is provided on the bottom housing of the device body, dust tends to gather in the bottom housing. In addition, the airbag occupies space in the bottom housing when the airbag is disposed on the bottom housing of the device body, and consequently, space inside the bottom housing is limited. In addition, the airbag and the watch strap are disposed separately. In a measurement process, a connection between the watch strap and the airbag is not attached to the wrist, causing uncomfortable wearing and affecting measurement accuracy.

To resolve at least one of the foregoing problems, embodiments of this application provide a wearable device. The wearable device includes a device body, at least one watch strap assembly, and a pressure measurement assembly. A vent is provided on a side wall of the device body, one of the at least one watch strap assembly includes a first watch strap body and an airbag, the airbag is detachably attached to an inner side of the first watch strap body, and one end of the airbag includes a first air nozzle, the first air nozzle is in communication with one end of an air flow channel that is of the first watch strap body and that is close to the device body, the other end of the air flow channel is in communication with the vent when the first watch strap body is connected to the side wall of the device body, and an end that is of the first watch strap body and that is connected to the device body covers the vent. In this way, when the wearable device is worn on a wrist, because the airbag is attached to the inner side of the first watch strap body, the first air nozzle of the airbag is connected to the air flow channel at one end of the first watch strap body, and the airbag and the first watch strap body are assembled into an overall structure and connected to the device body, the airbag can implement a good attaching function on the wrist after being inflated, so that wearing the airbag on the wrist is not uncomfortable in a wearing process and during blood pressure measurement of the wearable device. In this way, wear comfort of the wearable device in a wearing process and during blood pressure measurement is improved, and a problem of uncomfortable wearing of the wearable device is avoided. In addition, when one end of the first watch strap body that is provided with the air flow channel is connected to the device body, the end that is of the first watch strap body and that is connected to the side wall of the device body covers the vent on the side wall of the device body, so that the vent of the device body is not exposed, avoiding a problem that dust tends to gather at the vent of the wearable device. In addition, the airbag is located on the inner side of the first watch strap body, and does not occupy bottom housing space of the device body, so that the device body of the wearable device is thinner when the wearable device has a blood pressure measurement function. Therefore, the wearable device provided in embodiments of this application resolves problems that the wearable device is uncomfortable to wear during blood pressure measurement and dust tends to gather at the vent.

The following describes in detail a specific structure of the wearable device provided in embodiments of this application.

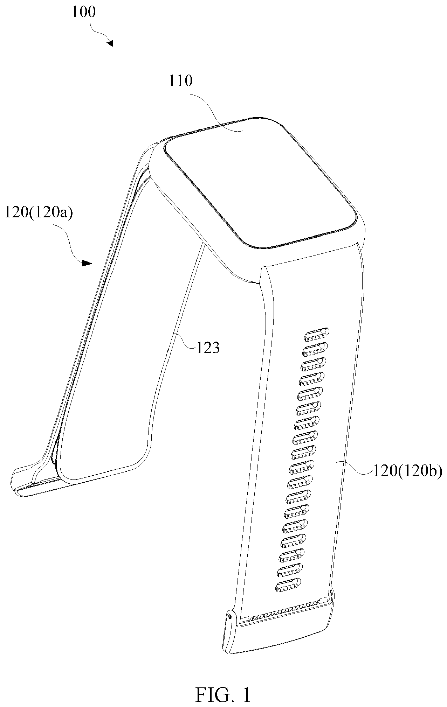

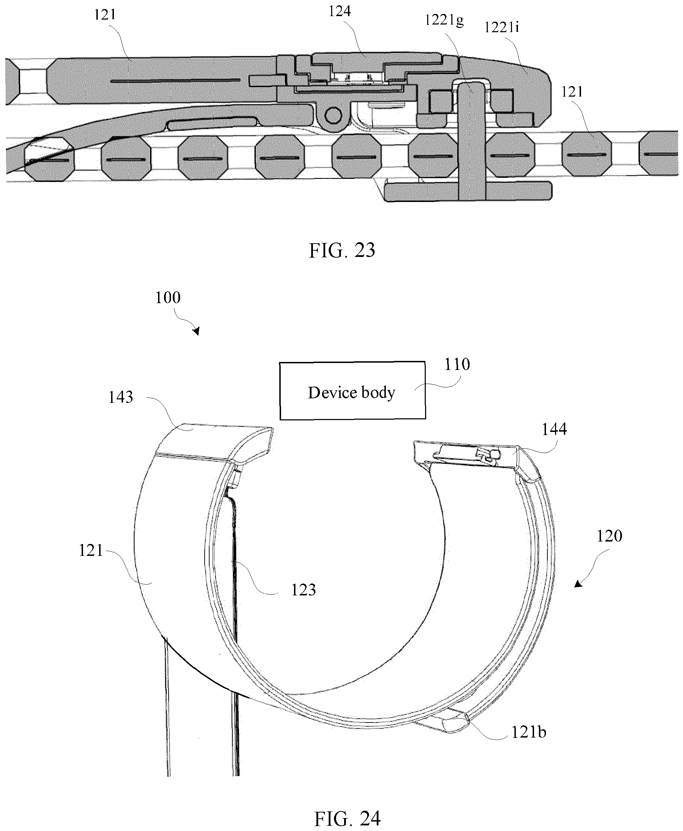

Refer to FIG. 1. A wearable device 100 provided in embodiments of this application may include a device body 110 and at least one watch strap assembly 120. For example, in FIG. 1, there are two watch strap assemblies 120, and the two watch strap assemblies 120 are respectively a first watch strap assembly 120a and a second watch strap assembly 120b. Certainly, in some examples, for example, there may be one watch strap assembly 120 (refer to FIG. 24 below). At least one end of each watch strap assembly 120 is connected to a side wall 111 of the device body 110. For example, in FIG. 1, one end of each watch strap assembly 120 is connected to the side wall 111 of the device body 110. In some examples, two ends of the watch strap assembly 120 are connected to the side walls 111 of the device body 110 (refer to FIG. 24 below).

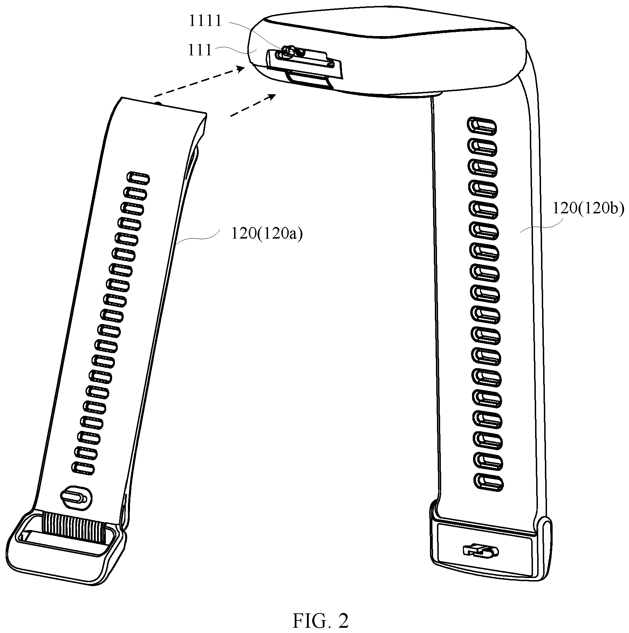

Refer to FIG. 2. The device body 110 includes the side wall 111, and a vent 1111 is provided on the side wall 111. The side wall 111 of the device body 110 may be of a ring-shaped middle frame structure. When there are two watch strap assemblies 120, the two watch strap assemblies 120 may be respectively connected to two side walls 111 of the device body 110 that are opposite to each other.

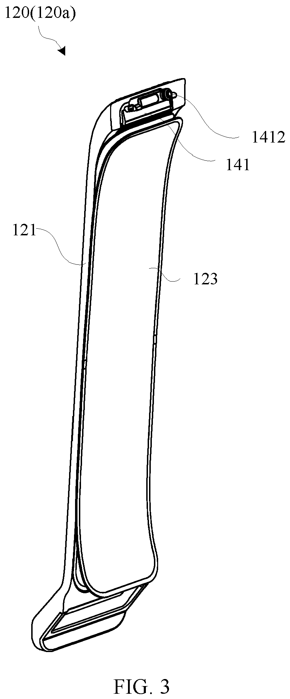

Refer to FIG. 3. One of the at least one watch strap assembly 120 may include: a first watch strap body 121 and an airbag 123. For example, there are two watch strap assemblies 120: a first watch strap assembly 120a and a second watch strap assembly 120b. Refer to FIG. 3. The first watch strap assembly 120a may include a first watch strap body 121 and an airbag 123. Refer to FIG. 3. The airbag 123 is detachably attached to an inner side of the first watch strap body 121 (that is, a side of the first watch strap body 121 that faces a wrist). One end of the first watch strap body 121 is connected to the device body 110. Certainly, the second watch strap assembly 120b may include the airbag 123, and one of the two watch strap assemblies 120 may include the airbag 123. In this embodiment of this application, an example in which the first watch strap assembly 120a includes an inflatable airbag 123 is specifically used for description. Certainly, in some examples, the airbags 123 may alternatively be disposed in both the first watch strap assembly 120a and the second watch strap assembly 120b. It should be noted that the airbag 123 in the watch strap assembly 120 may be in a bulged state when being inflated, so that blood pressure of a user can be measured.

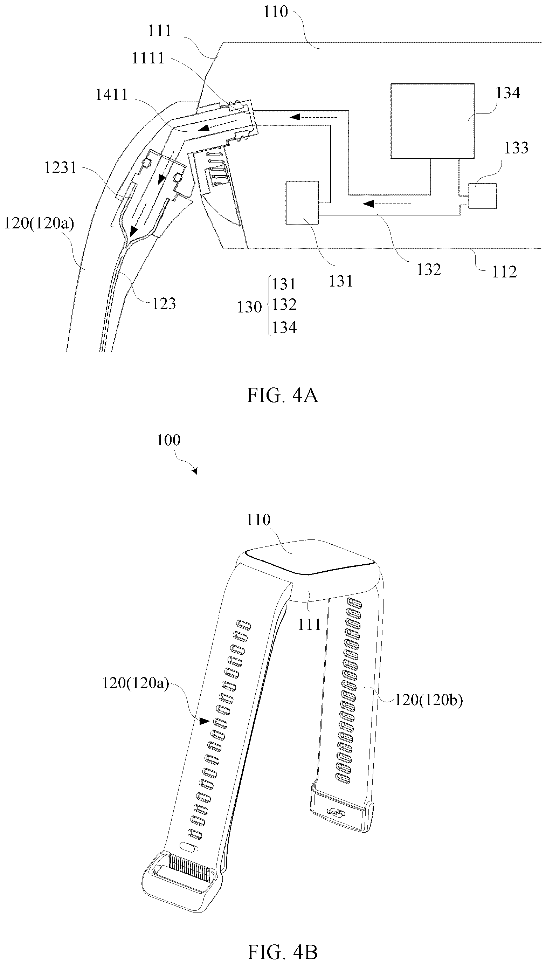

An inflatable air cavity is provided in the airbag 123. Refer to FIG. 4A. One end of the airbag 123 includes a first air nozzle 1231, an air flow channel 1411 is provided at one end of the first watch strap body 121, and the first air nozzle 1231 is in communication with one end of the air flow channel 1411. For example, the first air nozzle 1231 may be in communication with one end of the air flow channel 1411 in a detachable manner. In this way, the airbag 123 is connected to and in communication with the air flow channel 1411 of the first watch strap body 121, so that the airbag 123 and the first watch strap body 121 form the first watch strap assembly 120a (refer to FIG. 3) of an overall structure, and an inner side of the first watch strap assembly 120a is the airbag 123. When the airbag 123 is worn, the airbag 123 faces the wrist. During blood pressure measurement, the airbag 123 bulges, to implement a good attaching effect on the wrist, so that uncomfortable wearing of the wearable device does not occur between the airbag 123 and the wrist in a wearing process and during blood pressure measurement. This avoids a problem of uncomfortable wearing of the wearable device.

Refer to FIG. 4A. The other end of the air flow channel 1411 is in communication with the vent 1111 when the first watch strap body 121 is connected to the side wall of the device body 110. In this way, communication of the airbag 123 and the vent 1111 is implemented. Refer to FIG. 4A. In this embodiment of this application, to implement a blood pressure measurement function, the wearable device further includes: a pressure measurement assembly 130. The pressure measurement assembly 130 is disposed in the device body 110, the pressure measurement assembly 130 is in communication with the vent 1111, and the pressure measurement assembly 130 inflates the airbag 123 through the vent 1111.

Refer to FIG. 4A. The pressure measurement assembly 130 includes a pressure difference sensor 131, an air path 132, and an air pump 134. The vent 1111 is separately in communication with the pressure difference sensor 131 and the air pump 134 through the air path 132, and a control valve 133 is disposed on the air path 132. The control valve 133 may control a magnitude of an air flow on the air path 132. The air pump 134 may provide an air flow, and the air flow enters the airbag 123 through the air path 132. The pressure difference sensor 131 is configured to obtain a pressure difference of the air flow. The pressure difference sensor 131 may be electrically connected to a circuit board in the device body 110. The circuit board obtains a blood pressure value of the user based on a pressure difference signal obtained by the pressure difference sensor 131.

In this embodiment of this application, to ensure that dust does not tend to gather at the vent 1111 of the device body 110, the vent 1111 of the device body 110 is disposed invisible in appearance. Refer to FIG. 4A. When one end of the first watch strap assembly 120a is connected to the side wall 111 of the device body 110, the end that is of the first watch strap assembly 120a and that is connected to the device body 110 covers the vent 1111. In this way, as shown in FIG. 4B, when the first watch strap assembly 120a is connected to the side wall 111 of the device body 110, the airbag 123 is in communication with the vent 1111, and the vent 1111 of the device body 110 is invisible in appearance, to avoid a problem that dust gathers at the vent. In addition, the airbag 123 is located on the watch strap assembly 120, and does not occupy space in a bottom housing of the device body 110, so that the device body of the wearable device is thinner when the wearable device has a blood pressure measurement function. Therefore, the wearable device provided in this embodiment of this application resolves problems that the wearable device is uncomfortable to wear during blood pressure measurement and that dust tends to gather at the vent.

Therefore, according to the wearable device 100 provided in this embodiment of this application, the vent 1111 is disposed on the side wall 111 of the device body 110, one of the at least one watch strap assembly 120 includes the inflatable airbag 123, one end of the watch strap assembly 120 that includes the airbag 123 covers the vent 1111, and the vent 1111 is in communication with the airbag 123 through the air flow channel 1411 at one end of the first watch strap body 121, the inflatable airbag 123 is located on the watch strap assembly 120. When the watch strap assembly 120 is connected to the device body 110, in one aspect, the airbag 123 is in communication with the vent 1111, to implement a blood pressure measurement function, and in another aspect, one end of the watch strap assembly 120 covers the vent 1111 on the device body 110, so that dust does not tend to gather at the vent 1111 of the device body 110. In addition, the inflatable airbag 123 is located on the inner side of the first watch strap body 121. In this way, when blood pressure measurement is performed in a wearing process, the airbag 123 is well attached to the wrist, so that uncomfortable wearing does not occur between the airbag and the wrist in both the wearing process and the blood pressure measurement of the wearable device, improving wear comfort of the wearable device in the wearing process and the blood pressure measurement. Therefore, according to the wearable device provided in this embodiment of this application, this resolves a problem that wearing the wearable device is uncomfortable during blood pressure measurement and that dust tends to gather at the vent.

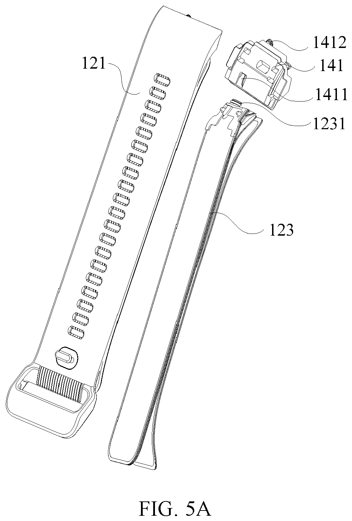

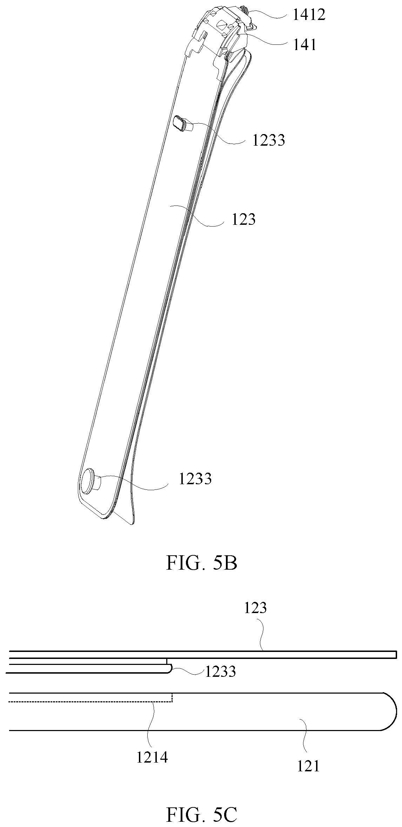

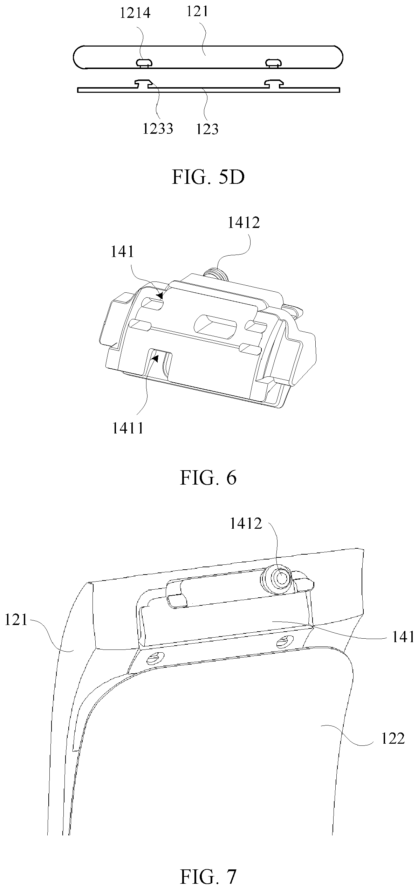

Refer to FIG. 5A. In this embodiment of this application, to implement that one end of the first watch strap assembly 120a is connected to the side wall 111 of the device body 110 one end of the first watch strap body 121 includes a first connection structure 141, an air flow channel 1411 is provided on the first connection structure 141 (as shown in FIG. 4A or FIG. 6 below), one end of the first connection structure 141 is connected to the first watch strap body 121, and the other end of the first connection structure 141 is detachably connected to the side wall 111 of the device body 110. Refer to FIG. 5A and FIG. 5B. The first air nozzle 1231 of the airbag 123 is connected to the first connection structure 141, and the first air nozzle 1231 is in communication with the vent 1111 through the air flow channel 1411. It should be noted that, in this embodiment of this application, when one end of the first connection structure 141 is connected to the first watch strap body 121, one end of the first connection structure 141 may be integrated with the first watch strap body 121 in a processing molding or assembling manner.

In this embodiment of this application, when the first watch strap body 121 is attached to the airbag 123, the first watch strap body 121 is attached to the airbag 123 in a fastening manner. Therefore, a fastening press strip 1233 is disposed on one of the first watch strap body 121 and the airbag 123, and a slot 1214 that is fastened to the fastening press strip 1233 is provided on the other one of the watch strap body 121 and the airbag 123. For example, refer to FIG. 5C. A fastening press strip 1233 is disposed on the airbag 123, and a slot 1214 that fits the fastening press strip 1233 is provided on the first watch strap body 121. Certainly, in some examples, a structure of the fastening press strip 1233 is not limited to a strip shape shown in FIG. 5C. For example, refer to FIG. 5D, the fastening press strip 1233 disposed on the airbag 123 may be mushroom-shaped, the first watch strap body 121 is provided with a slot 1214 that fits the mushroom-shaped fastening press strip 1233.

In this embodiment of this application, the first watch strap body 121 and the airbag 123 are fastened and fit with each other by using the fastening press strip 1233 and the slot 1214, so that the watch strap assembly 120 is well fit with the airbag 123, improving wearing comfort and occupying less space, and resolving a problem that the watch strap is not attached to the airbag 123 in a wearing process and causing uncomfortable wearing.

Refer to FIG. 6 and FIG. 7. In a possible implementation, the end of the first connection structure 141 that is connected to the device body 110 includes a second air nozzle 1412 in communication with the air flow channel 1411, and the air flow channel 1411 is connected to the vent 1111 in a sealed manner by using the second air nozzle 1412. For example, a sealing structure may be disposed around the second air nozzle 1412. In this way, after the second air nozzle 1412 is inserted into the vent 1111, the vent 1111 and the second air nozzle 1412 are connected in a sealed manner.

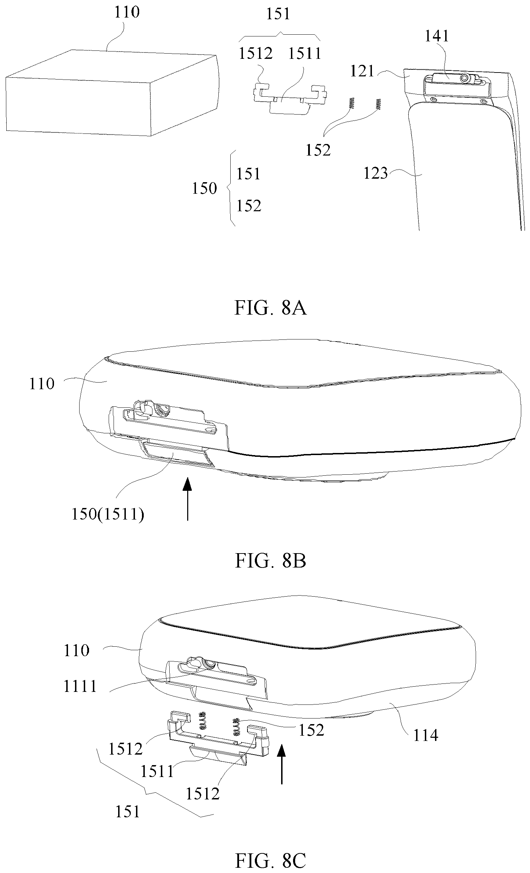

Refer to FIG. 8A. In a possible implementation, to implement a detachable connection between the first watch strap assembly 120a and the device body 110, the wearable device further includes: a first control button 150. Refer to FIG. 8B. The first control button 150 is disposed on the device body 110, and the first control button 150 is configured to control on connecting or disconnecting the first connection structure 141 and the device body 110. For example, when the first connection structure 141 is fastened to the device body 110, the first control button 150 locks a connection between the first connection structure 141 and the device body 110, so that the first connection structure 141 and the device body 110 cannot be detached. When the first watch strap assembly 120a needs to be detached from the device body 110, the first control button 150 is pressed, so that the connection between the first connection structure 141 and the device body 110 is unlocked. In this way, there is no connection between the first watch strap assembly 120a and the device body 110, and the first watch strap assembly 120a may be detached from the device body 110. In this way, in one aspect, the watch strap assembly 120 is detachably connected to the device body 110, and in another aspect, more functional watch straps can be compatible, so that the wearable device 100 becomes a multi-functional smart watch strap, and is not limited to blood pressure measurement.

Refer to FIG. 8A. When the first control button 150 controls the first connection structure 141 to be connected to or detached from the device body, specifically, the first control button 150 may include a button body 151 and an elastic part 152, and the button body 151 includes a pressing part 1511 and two fastening parts 1512 respectively located on two sides of the pressing part 1511. Refer to FIG. 8B. The pressing part 1511 of the first control button 150 is exposed on an outer surface of the device body 110, the pressing part 1511 is configured to move along a thickness direction of the device body (for example, an arrow direction in FIG. 8B) when being pressed. For example, when the user applies an acting force in the arrow direction in FIG. 8B to the pressing part 1511, the first control button 150 moves along the direction shown by the arrow, and the elastic part 152 is compressed. After the external force on the pressing part 1511 is removed, the pressing part 1511 is reset due to an elastic force of the elastic part 152. The fastening part 1512 and the elastic part 152 of the first control button 150 are located in the device body 110.

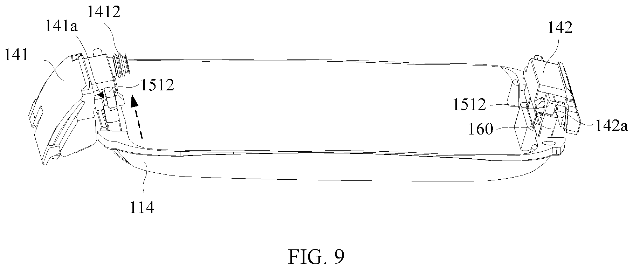

Refer to FIG. 8C. One end of the elastic part 152 is connected to the pressing part 1511, the other end of the elastic part 152 is connected to the device body 110, and the elastic part 152 is configured to enable the button body 151 to extend or retract on the device body 110. Refer to FIG. 8C and FIG. 8B. The pressing part 1511 (refer to FIG. 8C) of the button body 151 is located at an edge of the bottom housing 114 of the device body 110. Refer to FIG. 9. The fastening part 1512 is configured to fit a fastening slot 141a provided on the first connection structure 141, to connect the first connection structure to the device body 110. When the first watch strap assembly 120a needs to be detached from the device body 110, a force in an arrow direction shown in FIG. 9 is applied to the pressing part 1511. The pressing part 1511 moves in the arrow direction, and drives the fastening part 1512 to move in the arrow direction. In this way, the fastening part 1512 is detached from the fastening slot 141a on the first connection structure 141, the first control button 150 is detached from the first connection structure 141, and the first watch strap assembly 120a may be removed from the device body 110. After the external force on the pressing part 1511 is removed, an elastic force of the elastic part 152 drives the button body 151 to move in a direction opposite to the arrow direction to implement reset.

When the first connection structure 141 of the first watch strap assembly 120a is connected to the device body 110 again, the pressing part 1511 may be pressed again, so that the fastening part 1512 moves upward for a specific distance in the arrow direction. After one end of the fastening slot 141a provided on the first connection structure 141 extends into the device body 110, when an external force is applied to the pressing part 1511, the fastening part 1512 is fastened in the fastening slot 141a. A guide surface (not shown) may be provided on one end of the first connection structure 141 on which the fastening slot 141a is provided. When the first connection structure 141 extends into the device body 110, the fastening part 1512 is driven to move in the arrow direction until the fastening part 1512 is fastened in the fastening slot 141a, so that the first connection structure 141 is connected to the first control button 150.

In this embodiment of this application, the airbag 123 is disposed in the first watch strap assembly 120a, and no airbag 123 is disposed in the second watch strap assembly 120b. Refer to FIG. 9. The second watch strap assembly 120b includes a second watch strap body 122 and a watch buckle 1221. One end of the second watch strap body 122 is connected to the device body 110, the other end of the second watch strap body 122 is connected to the watch buckle 1221, and the watch buckle 1221 is configured to be detachably connected to one end of the first watch strap body 121.

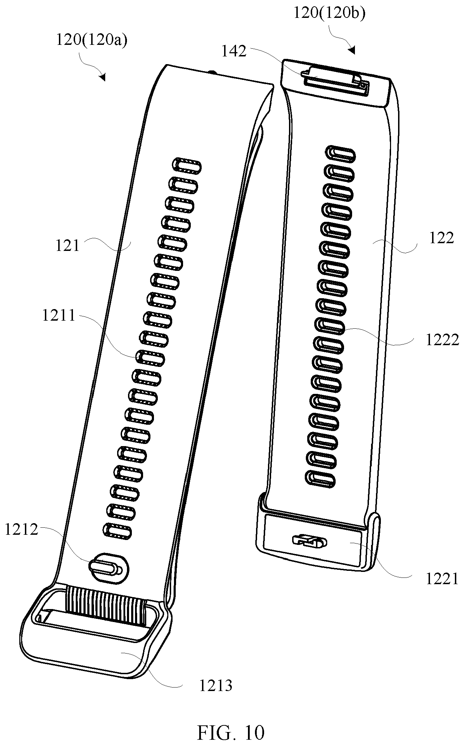

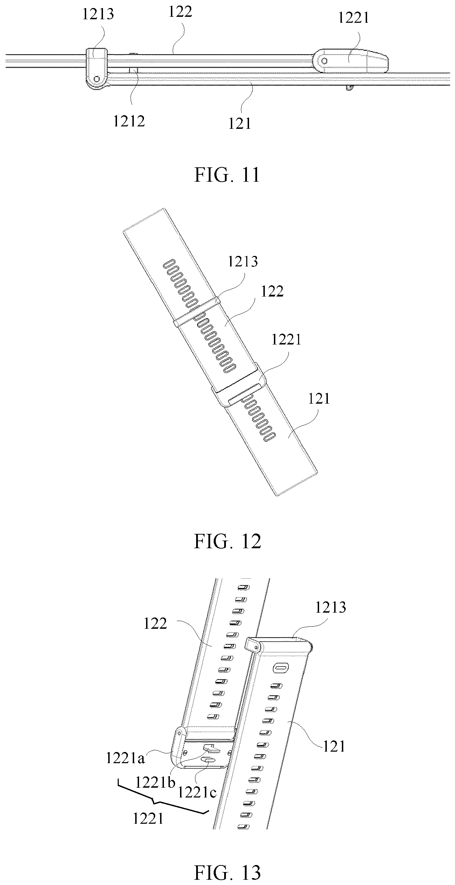

Refer to FIG. 10. During wearing, when the first watch strap body 121 and the second watch strap body 122 are detachably connected by using the watch buckle 1221, a plurality of first adjustment holes 1211 spaced apart are provided on the first watch strap body 121. Refer to FIG. 11. The first adjustment hole 1211 is configured to fasten the watch buckle 1221. In this way, after the first watch strap body 121 and the second watch strap body 122 are fastened, the second watch strap body 122 is located on an outer side of the first watch strap body 121, and the second watch strap body 122 does not cause interference to the airbag 123.

Refer to FIG. 10. In this embodiment of this application, to ensure that the airbag 123 has a larger attaching position with the wrist, a length of the first watch strap body 121 is greater than a length of the second watch strap body 122, and the airbag 123 extends from one end of the first watch strap body 121 to the other end of the first watch strap body 121. In this way, when the airbag 123 is worn on the wrist, because the length of the first watch strap body 121 is larger, the second watch strap body 122 cooperates with the first watch strap body 121, and the second watch strap body 122 is located on an outer side of the first watch strap body 121, the airbag 123 is attached to the wrist with a longer length, so that the airbag 123 is attached to the wrist with a larger area, ensuring accurate blood pressure detection.

Refer to FIG. 10. In this embodiment of this application, a loop 1213 that is capable of being passed through by the watch buckle 1221 is disposed at one end that is of the first watch strap body 121 and that is disposed back to back the device body 110, and the loop 1213 is rotatably connected to the first watch strap body 121. In this way, the watch buckle 1221 of the second watch strap body 122 may pass through the loop 1213 and be fastened to the first adjustment hole 1211 of the first watch strap body 121.

Refer to FIG. 10. In this embodiment of this application, to make fastening of the first watch strap body 121 and the second watch strap body 122 more stable, a buckle nail 1212 is disposed on an outer side surface (that is, a side facing back to back the airbag 123) of the first watch strap body 121, and a plurality of second adjustment holes 1222 spaced apart are disposed on the second watch strap body 122. Refer to FIG. 11. The buckle nail 1212 is configured to be fastened on the second adjustment hole 1222. Refer to FIG. 12. After the first watch strap body 121 and the second watch strap body 122 are fastened, the first watch strap body 121 is located on an inner side and is opposite to a wrist, and the second watch strap body 122 is located on an outer side.

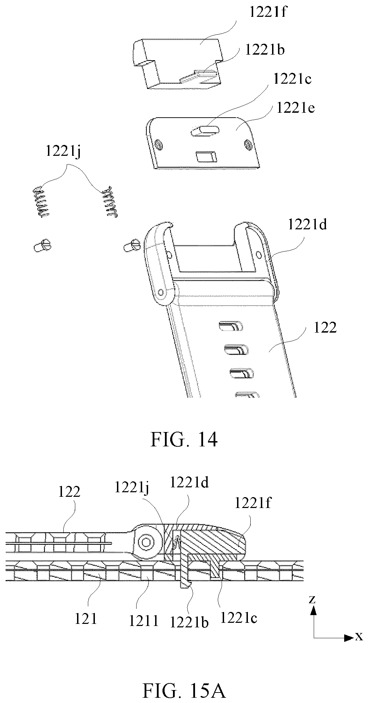

Refer to FIG. 13. In this embodiment of this application, for a structure of the watch buckle 1221. The watch buckle 1221 may include a watch buckle cover 1221a. A fastening post 1221c and a movable sliding buckle 1221b are disposed on the watch buckle cover 1221a. The fastening post 1221c is configured to fit one of the first adjustment holes 1211, and the sliding buckle 1221b is configured to be fastened to another first adjustment hole 1211. Refer to FIG. 14. The watch buckle cover 1221a may include an upper housing 1221d and a lower housing 1221e, where the fastening post 1221c is disposed on the lower housing 1221e, a movable sliding block 1221f is disposed between the upper housing 1221d and the lower housing 1221e, one end of the sliding block 1221f is provided with the sliding buckle 1221b, and the sliding block 1221f is slidably disposed in the watch buckle cover 1221a by using a spring 1221j.

Refer to FIG. 15A. During wearing, first, one end of the sliding block 1221f may be pushed (moved in a direction of -x), the spring 1221j is compressed, and the sliding block 1221f drives the sliding buckle 1221b to move leftward, so that the sliding buckle 1221b may extend into the first adjustment hole 1211 of the first watch strap, and the sliding block 1221f is released. The sliding block 1221f moves in a direction of x under an elastic force of the spring 1221j, the sliding buckle 1221b moves rightward, and an end part of the sliding buckle 1221b is fastened at an edge of the first adjustment hole 1211. In this way, the sliding buckle 1221b implements a limiting function on the first watch strap body 121 in the x direction and a z direction. The fastening post 1221c extends into another first adjustment hole 1211, and the fastening post 1221c plays a positioning role in the x direction. In this embodiment of this application, a double positioning function in the x direction is implemented by using the fastening post 1221c and the movable sliding buckle 1221b, so that a stable fastening effect exists between the watch buckle 1221 and the first watch strap body 121.

Therefore, the fastening post 1221c and the movable sliding buckle 1221b in the watch buckle 1221 are disposed, so that the watch buckle 1221 is opened and closed in a minimum space. In addition, convenience of opening and closing the two watch strap bodies and an attractive design of the watch buckle 1221 are implemented.

In this embodiment of this application, both the first watch strap body 121 and the second watch strap body 122 may be made of flexible plastic materials. For example, both the first watch strap body 121 and the second watch strap body 122 are plastic straps.

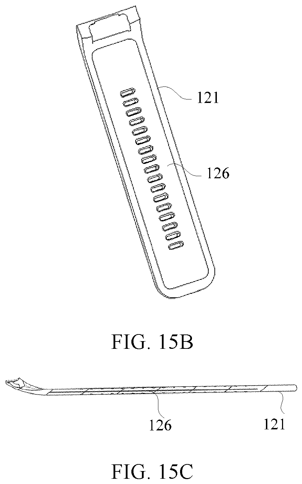

When both the first watch strap body 121 and the second watch strap body 122 are plastic straps, in a blood pressure measurement process, stretching deformation of the watch strap body greatly affects the airbag 123. Therefore, in an embodiment of this application, a tensile interlayer 126 is disposed on inner sides of the first watch strap body 121 and the second watch strap body 122. For example, as shown in FIG. 15B, a tensile interlayer 126 is disposed on an inner side of the first watch strap body 121, when the airbag 123 is attached to the inner side of the first watch strap assembly 120a, the tensile interlayer 126 is located between the first watch strap assembly 120a and the airbag 123. Alternatively, in some examples, the tensile interlayer 126 may be disposed in the first watch strap body 121 and the second watch strap body 122. For example, refer to FIG. 15C. The tensile interlayer 126 is disposed in the first watch strap body 121.

The tensile interlayer 126 is disposed in the watch strap assembly 120. In this way, when the first watch strap body 121 and the second watch strap body 122 are plastic straps, in a blood pressure measurement process, when the first watch strap body 121 and the second watch strap body 122 are stretched, the airbag 123 is not affected, ensuring accuracy of blood pressure measurement.

It should be noted that the tensile interlayer 126 may be an interlayer made of a material with high tensile strength. For example, the material with high tensile strength may be polyimide (Polyimide, PI), or the material of high tensile strength may be nylon.

When the tensile interlayer 126 is disposed in the first watch strap body 121 and the second watch strap body 122, the first watch strap body 121 and the second watch strap body 122 may be integrated with a material of high tensile strength in a molding process. For example, a material of the first watch strap body 121 and the material of the second watch strap body 122 may be a plastic material. The plastic material is first molded. During the first molding, a middle plate is placed in the plastic material of two pieces and a positioning post is placed on the middle plate, used for positioning a film material; and after the first molding, the middle plate is taken out from the plastic material, and then the second molding is performed on the plastic material, to integrate the material with high tensile strength into the plastic material. A tensile capability of the first watch strap body 121 and the second watch strap body 122 that are formed are greatly improved. When the first watch strap body 121 and the second watch strap body 122 are stretched, the tensile interlayer 126 in the first watch strap body 121 and the second watch strap body 122 bear a tensile force, so that deformation of the first watch strap body 121 and the second watch strap body 122 is controlled within a small range.

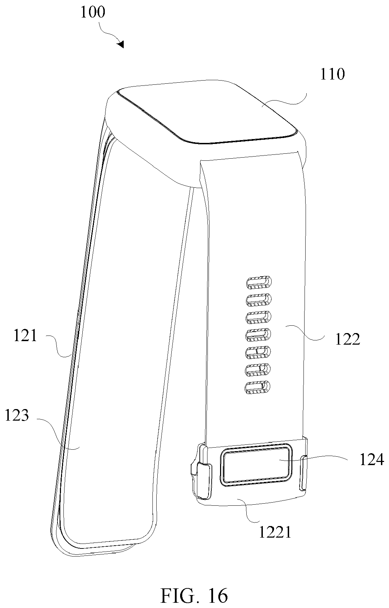

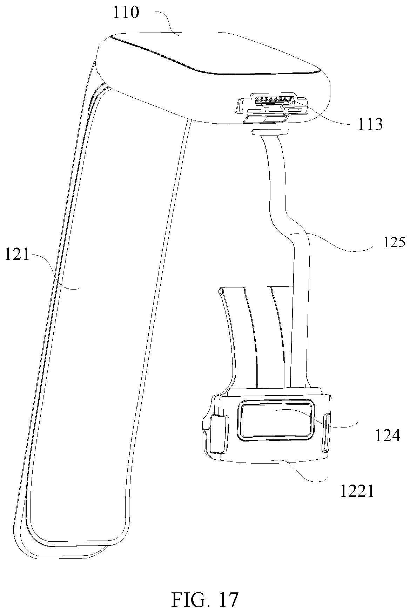

In this embodiment of this application, in addition to a blood pressure detection function of the wearable device 100, another detection function may be further configured on the wearable device 100. For example, as shown in FIG. 16, a detection electrode 124 is further disposed on the watch buckle 1221, and a flexible circuit board 125 is disposed in the second watch strap body 122. Refer to FIG. 17. The detection electrode 124 is electrically connected to a circuit board (not shown) in the device body 110 by using the flexible circuit board 125. The detection electrode 124 may be disposed on the watch buckle 1221 in an exposed manner. The detection electrode 124 may be a sensor, and the detection electrode 124 may be attached to a leg or a chest of a human body to complete measurement of a heartbeat, body fat, or the like. In this embodiment of this application, the detection electrode 124 is disposed on the watch buckle 1221, so that a function of the wearable device 100 is added, so that a human body is in contact with a sensor, and electrocardiogram measurement precision is improved.

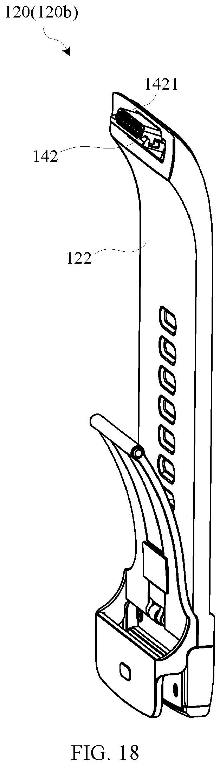

Refer to FIG. 18. In this embodiment of this application, to connect the second watch strap body 122 and the side wall 111 of the device body 110, a second connection structure 142 is further included. One end of the second connection structure 142 is connected to the second watch strap body 122, and the other end of the second connection structure 142 is connected to the device body 110.

To implement detachable connection between the second watch strap body 122 and the device body 110, the device further includes a second control button 160 (refer to FIG. 9). The second control button 160 is disposed on the device body 110, and the second control button 160 is configured to control the second connection structure 142 to be connected to or detached from the device body 110. For a structure and a function principle of the second control button 160, refer to the first control button 150 in FIG. 8A. For example, the second control button 160 may include a button body 151 and an elastic part 152, and the button body 151 includes a pressing part 1511 and two fastening parts 1512 respectively located on two sides of the pressing part 1511. The second connection structure 142 is provided with a fastening slot 142a that cooperates with the fastening part 1512 of the second control button 160. For cooperation between the second control button 160 and the second connection structure 142, refer to the first control button 150 and the first connection structure 141. Details are not described again in this embodiment of this application.

Refer to FIG. 18. When the detection electrode 124 is disposed on the watch buckle 1221, to implement signal transmission between the detection electrode 124 and the device body 110, a conductive contact 1421 is disposed on the second connection structure 142, the conductive contact 1421 is electrically connected to the flexible circuit board 125, a conductive terminal 113 (refer to FIG. 17) in electrical contact with the conductive contact 1421 is disposed on a surface that is of the device body 110 and that is connected to the second connection structure 142, and the conductive terminal 113 is electrically connected to the circuit board in the device body 110. In this way, after the second watch strap body 122 is connected to the device body 110 by using the second connection structure 142, the conductive terminal 113 is connected to the conductive contact 1421, and the detection electrode 124 is connected to the circuit board in the device body 110 by using the flexible circuit board 125, implementing signal transmission.

It should be noted that, in this embodiment of this application, in FIG. 17, the detection electrode 124 is disposed on the watch buckle 1221. In some examples, other detection electrodes 124 may be further disposed on the side wall 111 and the bottom wall 112 of the device body 110. In this way, a function of the wearable device 100 can be added.

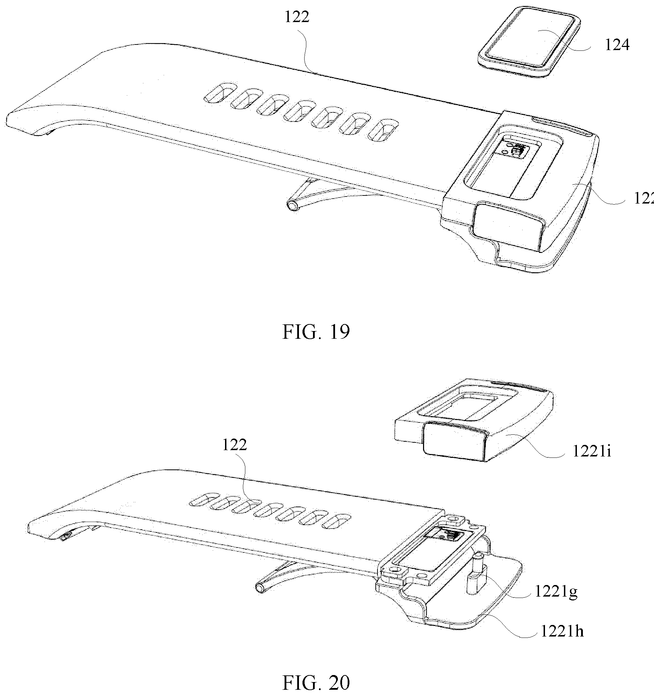

Refer to FIG. 19. When the detection electrode 124 is disposed on the watch buckle 1221, a mounting slot may be provided on a surface of the watch buckle 1221, and the detection electrode 124 is disposed in the mounting slot.

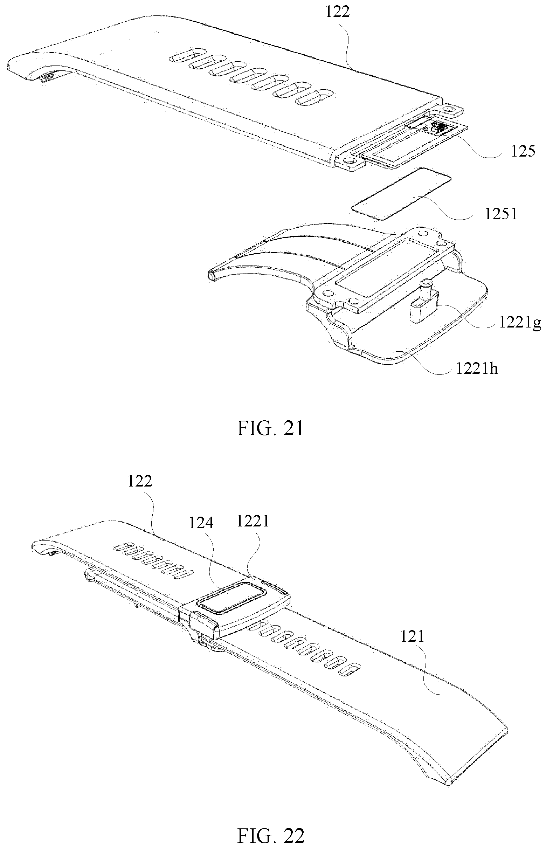

In an embodiment of this application, another structure of the watch buckle 1221 may be shown in FIG. 20. The watch buckle 1221 may include: a base 1221h and an upper cover 1221i. The upper cover 1221i is rotatably connected to the base 1221h. A gap that is capable of being passed through by one end of the first watch strap body 121 is provided between the upper cover 1221i and the base 1221h. A buckle pin 1221g for fastening to the first adjustment hole 1211 is disposed on a surface that is of the upper cover 1221i and that faces the base 1221h. The detection electrode 124 is disposed on the upper cover 1221i. Refer to FIG. 21. One end of the flexible circuit board 125 may be fastened to the bottom housing by using an adhesive layer 1251. After the upper cover 1221i is assembled with the base 1221h, the flexible circuit board 125 is electrically connected to the detection electrode 124 by using a spring or the like.

Refer to FIG. 22. One end of the first watch strap body 121 passes through the watch buckle 1221, the second watch strap body 122 is located on the outer side, the first watch strap body 121 is located on the inner side, and the detection electrode 124 is located on an outward-facing surface of the watch buckle 1221. It should be noted that, when the airbag 123 is disposed on the inner side of the first watch strap body 121, to avoid affect caused by the base 1221h of the watch buckle 1221 on the airbag 123, a length of the airbag 123 is limited, to ensure that the airbag 123 does not extend to the watch buckle 1221 when the watch buckle 1221 is fastened to the first watch strap body 121.

It should be noted that, when a structure of the watch buckle 1221 provided in this embodiment of this application is shown as a structure in FIG. 22, a loop 1213 may not be disposed at an end that is of the first watch strap body 121 and that is not connected to the device body 110.

Refer to FIG. 23. After the first watch strap body 121 passes through the watch buckle 1221, the buckle pin 1221g may be fastened in the first adjustment hole 1211 of the first watch strap body 121. In this way, the watch buckle 1221 is fastened to the first watch strap body 121.

The following describes another structure of the wearable device 100 provided in an embodiment of this application.

Refer to FIG. 24. A difference between this embodiment of this application and the foregoing embodiments is as follows: In this embodiment of this application, there is one watch strap assembly 120, for example, only a first watch strap assembly 120a. Refer to FIG. 24. The first watch strap assembly 120a includes a first watch strap body 121 and an airbag 123. Two ends of the first watch strap body 121 are connected to a device body 110. It should be noted that the airbag 123 in FIG. 24 is not attached to the first watch strap body 121.

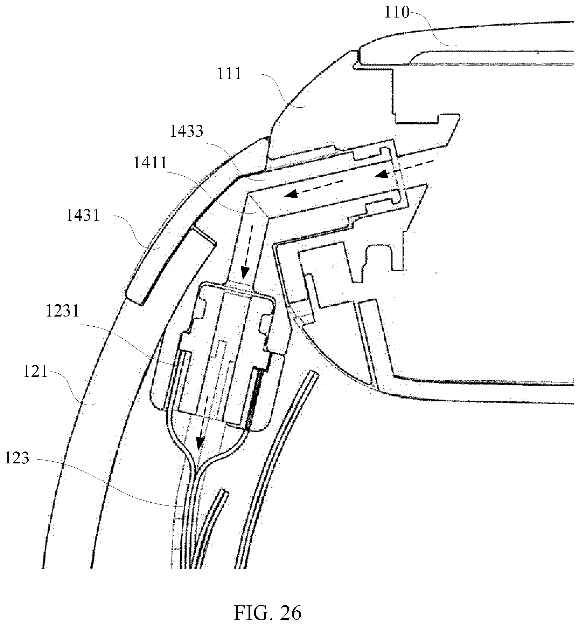

Refer to FIG. 24. In this embodiment of this application, a first end 121a of the first watch strap body 121 is connected to a side wall 111 of the device body 110, a second end 121b of the first watch strap body 121 is in sliding cooperation with the device body 110, and the second end 121b of the first watch strap body 121 is detachably connected to the first watch strap body 121. In this embodiment of this application, the airbag 123 may extend along one end of the first watch strap body 121 to the other end of the first watch strap body 121. In this scenario, after the airbag 123 is inflated, a sliding position of the first watch strap body 121 and the device body 110 do not squeeze the airbag 123. For example, after the airbag 123 is inflated, the airbag 123 may be in contact with the sliding position of the device body 110 and the first watch strap body 121, but the airbag 123is not squeezed or touched. Alternatively, in some examples, a length of the airbag 123 may be less than a length of the first watch strap body 121. For example, one end of the airbag 123 is connected to one end (refer to the following FIG. 26) that is of the first watch strap body 121 and at which the air flow channel 1411 is provided, and the other end of the airbag 123 extends to an inner side of the sliding connection between the first watch strap body 121 and the device body 110 in FIG. 24. It should be noted that, because wrists of users are different in thickness, therefore, sliding lengths of the second end 121b of the first watch strap body 121 are different. To ensure that the airbag 123 does not slide out from an inner side of the first watch strap body 121 to an outer side of the first watch strap body 121, the length of the airbag 123 is set as long as a measurement requirement is met. For example, when the second end 121b of the first watch strap body 121 slides to a maximum sliding amount, a length between the first end 121a of the first watch strap body 121 and the sliding connection position of the first watch strap body 121 and the device body 110 may be a length of the airbag 123.

In this embodiment of this application, the first watch strap body 121 may be a non-elastic woven strap. For example, the first watch strap body 121 may be woven by using a ribbon weaving machine, and the first watch strap body 121 may be of a double-sided terry structure. Elastic force of the first watch strap assembly 120a may be controlled by adding thermal fuse by proportion, through a fabric design of yarn, and by adding sewing threads on two edges of the woven strap.

In this embodiment of this application, the airbag 123 may be of a two-layer or multi-layer structure. A side that is of the airbag 123 and that is opposite to the wrist may be made of a two-layer nylon-TPU (thermally plastic polyurethane) composite film material, and has an antibacterial function. A side that is of the airbag 123 and that is opposite to the first watch strap body 121 may be made of a two-layer or multi-layer nylon-TPU composite film material. The airbag 123 and the first watch strap body 121 may be connected by using a hook and loop fastener, for example, the hook and loop fastener may first be connected to the airbag 123 through laser welding, and then be fastened to the first watch strap body 121 by using a hook ring.

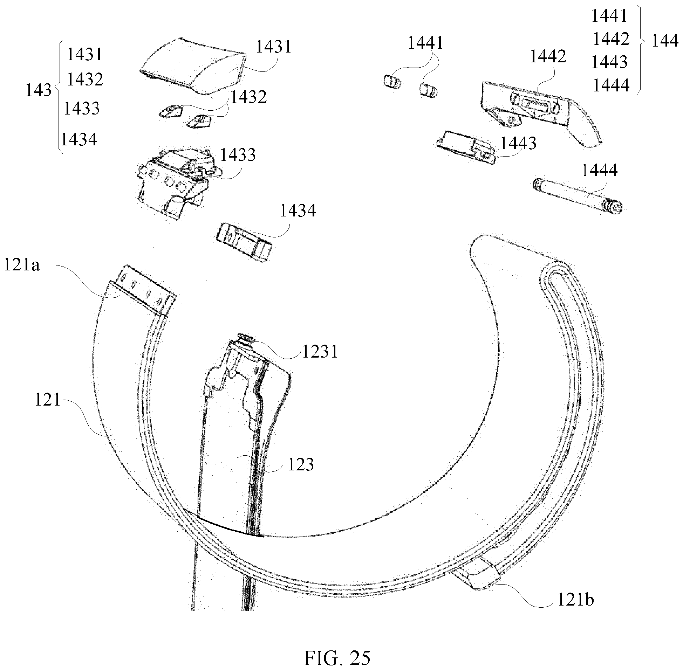

Refer to FIG. 25. In this embodiment of this application, to connect the first end 121a of the first watch strap assembly 120a and the device body 110, the first end 121a of the first watch strap body 121 includes a third connection structure 143, and the third connection structure 143 includes a first lug 1431 and a first end link 1433. Refer to FIG. 26. An air flow channel 1411 is provided in the first end link 1433, and one end of the first end link 1433 is connected to the first end 121a of the first watch strap body 121, the other end of the first end link 1433 is connected to the side wall 111 of the device body 110, and the airbag 123 is in communication with the vent 1111 through the air flow channel 1411. Refer to FIG. 26. The first lug 1431 is disposed to cover the first end link 1433, and the first lug 1431 is locked with the first end link 1433 by using a screw or the like. In this way, the first watch strap body 121 is connected to the device body 110 by using the first end link 1433, and the airbag 123 is in communication with the vent 1111 through the air flow channel 1411 in the first end link 1433. The first lug 1431 covers the first end link 1433 and a connection between the first end link 1433 and the first watch strap body 121, to ensure attractive appearance of the first watch strap assembly 120a.

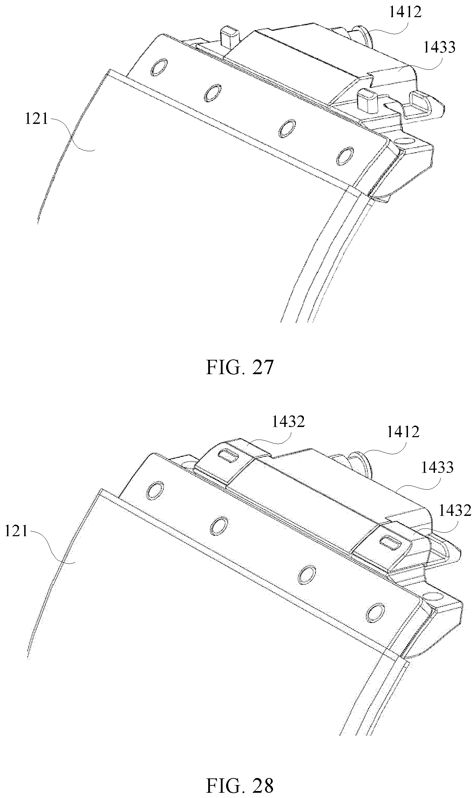

Refer to FIG. 27. In this embodiment of this application, when a head part of the first watch strap body 121 is connected to the first end link 1433, the head part of the first watch strap body 121 may be laser cut or punched to form a hole for connecting to the first end link 1433, and the head part of the first watch strap body 121 is hung on a protrusion on the first end link 1433.

Refer to FIG. 25 and FIG. 28. In this embodiment of this application, the first end link 1433 and the device body 110 are usually made of a metal material. To avoid a problem like deformation caused by touching each other by the first lug 1431 and the device body 110 when the first lug 1431 and the device body 110 are connected, in this embodiment of this application, the third connection structure 143 further includes: a first buffer body 1432. The first buffer body 1432 is disposed on the first end link 1433. For example, in FIG. 28, the first buffer body 1432 is fastened on the first end link 1433 by using a guide post on the first end link 1433, the first buffer body 1432 is located between the first lug 1431 and the side wall 111 of the device body 110, and the first buffer acts as a buffer when the first lug 1431 is connected to the device body 110.

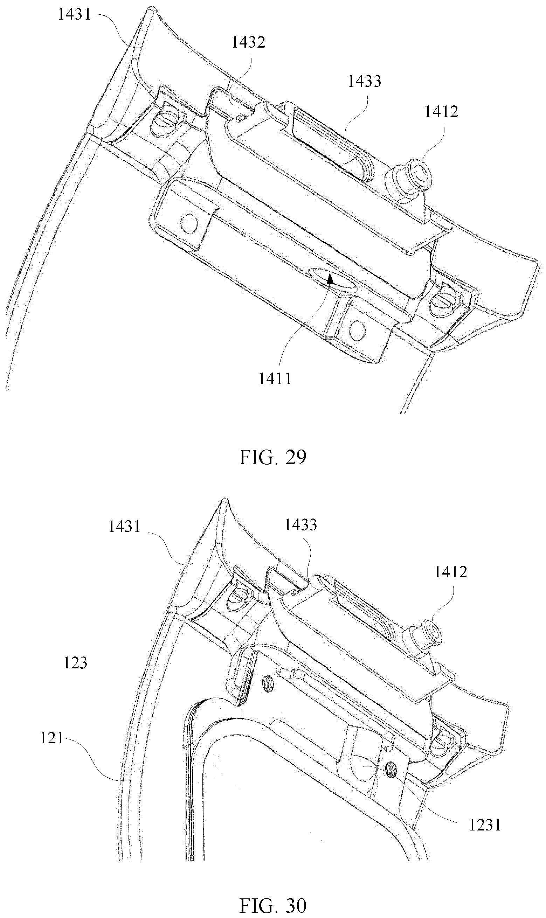

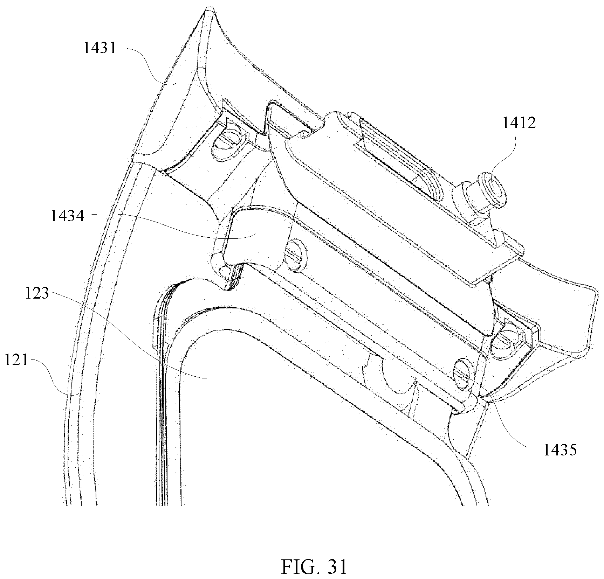

Refer to FIG. 28 and FIG. 29. When the third connection structure 143 is assembled, after the first buffer body 1432 is disposed on the first end link 1433, the first lug 1431 is installed on the first end link 1433, and the first lug 1431 and the first end link 1433 are locked by using a screw. Refer to FIG. 30. An end of the airbag 123 at which the first air nozzle 1231 is disposed extends into one end of the first end link 1433, so that the first air nozzle 1231 is connected to the air flow channel 1411 of the first end link 1433. Refer to FIG. 31. To fasten the airbag 123 to the first end link 1433, the third connection structure 143 further includes a cover plate 1434 and a fastener 1435 (which may be, for example, a screw or a bolt). The cover plate 1434 is pressed on the first air nozzle 1231 of the airbag 123, and the fastener 1435 is configured to fasten the cover plate 1434 to the first end link 1433, so that the airbag 123 is fastened to the first end link 1433.

Still refer to FIG. 25. In this embodiment of this application, to implement a sliding connection between the second end 121b of the first watch strap body 121 and the device body 110, the wearable device further includes a fourth connection structure 144. One end of the fourth connection structure 144 is connected to the device body 110 (as shown in FIG. 24), and the other end of the fourth connection structure 144 is slidably connected to the first watch strap body 121. The second end 121b of the fourth connection structure 144 may be connected to the first watch strap body 121 after passing through the fourth connection structure 144.

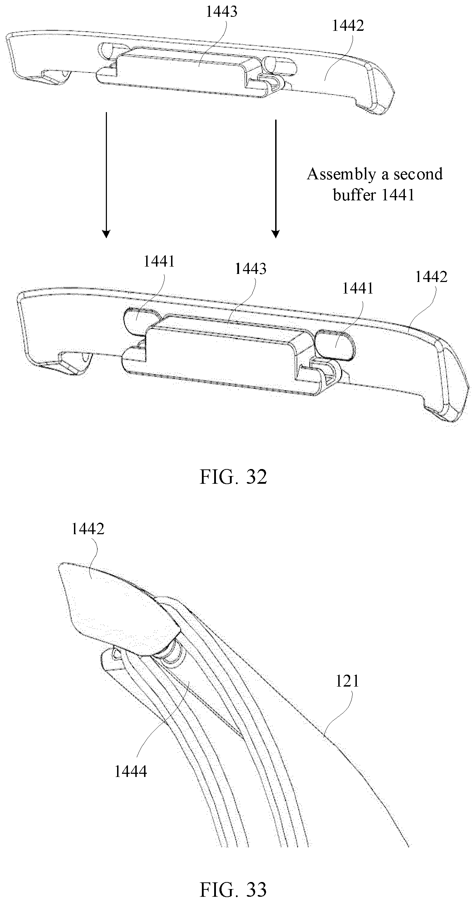

Refer to FIG. 25. The fourth connection structure 144 may include a second lug 1442, a second end link 1443, and a spring bar 1444. Refer to FIG. 32. During assembly, the second end link 1443 is connected to the second lug 1442, to form an overall structure.

Refer to FIG. 32. The fourth connection structure 144 further includes a second buffer body 1441. The second buffer body 1441 is disposed on the second lug 1442, and the second buffer body 1441 is located between the second lug 1442 and the side wall 111 of the device body 110. The second buffer body 1441 acts as a buffer between the second lug 1442 and the device body 110, to avoid deformation caused by mutual touching by the second lug 1442 and the device body 110 during assembly.

Refer to FIG. 33. Two ends of the spring bar 1444 are connected to the second lug 1442, sliding space for being passed through by the second end 121b of the first watch strap body 121 is provided between the spring bar 1444 and the second lug 1442, and the second end 121b of the first watch strap body 121 is slidably disposed around the spring bar 1444. In this way, when the first watch strap assembly 120a needs to be adjusted, for example, tightened or loosened, the second end 121b of the first watch strap body 121 may be pulled to move toward the first end 121a of the first watch strap body 121 (refer to FIG. 24), and the first watch strap body 121 slides around the spring bar 1444. When the first watch strap assembly 120a is adjusted, for example, tightened or loosened, the second end 121b of the first watch strap body 121 may be pulled to any length for adjustment, the first watch strap body 121 has no length limitation during tightening or loosening adjustment. Therefore, compared with adjustment step by step of a watch strap in the conventional technology, in this embodiment of this application, stepless adjustment during tightening or loosening of the first watch strap assembly 120a is implemented through sliding cooperation between the fourth connection structure 144 and the first watch strap body 121.

In this embodiment of this application, the spring bar 1444 may be a rotating shaft, and two ends of the spring bar 1444 may be connected to the second lug 1442 through rotation. In this way, when the second end 121b of the first watch strap body 121 is pulled, the first watch strap body 121 moves around the spring bar 1444, and the spring bar 1444 rotates, reducing mutual friction between the first watch strap body 121 and the spring bar 1444.

In this embodiment of this application, after the first watch strap body 121 is adjusted, for example, tightened or loosened, to fasten the second end 121b of the first watch strap body 121, a bonding component is disposed on the second end 121b of the first watch strap body 121, and a connecting component that is bonded to the bonding component is disposed on a side that is of the first watch strap body 121 and that is disposed back to back the airbag 123. The bonding component and the connecting component may be a pair of hook and loop fasteners that can be bonded to each other and can be torn apart. When the second end 121b of the first watch strap body 121 is adjusted to a specific position based on wrist thickness, the second end 121b is connected to an outer side surface of the first watch strap body 121 by using the bonding component and the connecting component, so that the first watch strap body 121 is fastened tightly or loosely. During unfastening, the second end 121b of the first watch strap body 121 may be pulled to detach from the first watch strap body 121.

An embodiment of this application may further provide a wearable system. The wearable system includes a terminal device and a wearable device 100. The terminal device is communicatively connected to the wearable device 100. The terminal device may be a smartphone, a tablet computer, or the like. The terminal device and the wearable device 100 may communicate with each other through Bluetooth or Wi-Fi.

Finally, it should be noted that the foregoing embodiments are merely intended for describing the technical solutions in embodiments of this application, instead of limiting the technical solutions. Although embodiments of this application are described in detail with reference to the foregoing embodiments, persons of ordinary skill in the art should understand that they may still make modifications to the technical solutions described in the foregoing embodiments or make equivalent replacements to some or all technical features thereof, without departing from the scope of the technical solutions in embodiments of this application.

## Claims

1. A wearable device, comprising:
a device body, wherein the device body comprises a side wall, and a vent is provided on the side wall;
at least one watch strap assembly, wherein at least one end of each watch strap assembly is connected to the side wall of the device body, one of the at least one watch strap assembly comprises a first watch strap body and an airbag, the airbag is detachably attached to an inner side of the first watch strap body, one end of the airbag comprises a first air nozzle, an air flow channel is provided at one end of the first watch strap body, the first air nozzle is in communication with one end of the air flow channel, the other end of the air flow channel is in communication with the vent when the first watch strap body is connected to the side wall of the device body, and an end that is of the first watch strap body and that is connected to the device body covers the vent; and
a pressure measurement assembly, wherein the pressure measurement assembly is disposed in the device body, and the pressure measurement assembly is in communication with the vent, to enable the pressure measurement assembly to inflate the airbag through the vent.

2. The wearable device according to claim 1, wherein there are two watch strap assemblies, and the two watch strap assemblies are a first watch strap assembly and a second watch strap assembly;
the first watch strap assembly comprises the first watch strap body and the airbag; and
the second watch strap assembly comprises a second watch strap body and a watch buckle, one end of the second watch strap body is connected to the device body, the other end of the second watch strap body is connected to the watch buckle, and the watch buckle is configured to be detachably connected to one end of the first watch strap body.

3. The wearable device according to claim 2, wherein one end of the first watch strap body comprises a first connection structure, and the first connection structure is provided with the air flow channel; and
the first air nozzle is connected to one end of the first connection structure, and the other end of the first connection structure is detachably connected to the side wall of the device body.

4. The wearable device according to claim 3, wherein an end that is of the first connection structure and that is connected to the device body comprises a second air nozzle that is in communication with the air flow channel, and the second air nozzle is connected to the vent in a sealed manner.

5. The wearable device according to claim 3 or 4, further comprising: a first control button, wherein the first control button is disposed on the device body, and the first control button is configured to control the first connection structure to be connected to or detached from the device body.

6. The wearable device according to claim 5, wherein the first control button comprises a button body and an elastic part, and the button body comprises a pressing part and two fastening parts respectively located on two sides of the pressing part;
the fastening part is configured to fit a fastening slot provided on the first connection structure, to connect the first connection structure to the device body;
the pressing part is configured to move in a thickness direction of the device body when being pressed, to enable the fastening part to be detached from the fastening slot; and
one end of the elastic part is connected to the pressing part, the other end of the elastic part is connected to the device body, and the elastic part is configured to enable the button body to extend or retract on the device body.

7. The wearable device according to any one of claims 2 to 6, wherein
a plurality of first adjustment holes spaced apart are provided on the first watch strap body, and the first adjustment hole is configured to fasten the watch buckle.

8. The wearable device according to claim 7, wherein a buckle nail is disposed on an outer side surface of the first watch strap body, a plurality of second adjustment holes spaced apart are provided on the second watch strap body, and the buckle nail is configured to be fastened and connected to the second adjustment hole.

9. The wearable device according to claim 7 or 8, wherein a loop that is capable of being passed through by the watch buckle is disposed at one end that is of the first watch strap body and that is disposed back to back the device body, and the loop is rotatably connected to the first watch strap body.

10. The wearable device according to any one of claims 7 to 9, wherein the watch buckle comprises a watch buckle cover, a fastening post and a movable sliding buckle are disposed on the watch buckle cover, the fastening post is configured to fit one of the first adjustment holes, and the sliding buckle is configured to be fastened to another one of the first adjustment holes.

11. The wearable device according to claim 7 or 8, wherein the watch buckle comprises a base and an upper cover, the upper cover is rotatably connected to the base, and a gap that is capable of being passed through by one end of the first watch strap body is provided between the upper cover and the base; and
a buckle pin for fastening to the first adjustment hole is disposed on a surface that is of the upper cover and that faces the base.

12. The wearable device according to any one of claims 2 to 11, wherein a detection electrode is further disposed on the watch buckle, a flexible circuit board is disposed in the second watch strap body, and the detection electrode is electrically connected to a circuit board in the device body through the flexible circuit board.

13. The wearable device according to claim 12, further comprising: a second connection structure and a second control button, wherein one end of the second connection structure is connected to the second watch strap body, the other end of the second connection structure is connected to the device body, a conductive contact is disposed on the second connection structure, the conductive contact is electrically connected to the flexible circuit board, and a conductive terminal in electrical contact with the conductive contact is disposed on a surface that is of the device body and that is connected to the second connection structure; and
the second control button is disposed on the device body, and the second control button is configured to control the second connection structure to be connected to or detached from the device body.

14. The wearable device according to any one of claims 2 to 13, wherein the first watch strap body and the second watch strap body are both of a flexible plastic material.

15. The wearable device according to claim 14, wherein a tensile interlayer is disposed in the first watch strap body and the second watch strap body, or is disposed on inner sides of the first watch strap body and the second watch strap body.

16. The wearable device according to any one of claims 2 to 15, wherein a fastening press strip is disposed on one of the first watch strap body and the airbag, and a slot that fits the fastening press strip is provided on the other one of the watch strap body and the airbag.

17. The wearable device according to any one of claims 2 to 16, wherein a length of the first watch strap body is greater than a length of the second watch strap body, and the airbag extends along one end of the first watch strap body to the other end of the first watch strap body.

18. The wearable device according to claim 1, wherein there is one watch strap assembly, a first end of a first watch strap body of the watch strap assembly is connected to the side wall of the device body, a second end of the first watch strap body is in sliding cooperation with the device body, and the second end of the first watch strap body is detachably connected to the first watch strap body.

19. The wearable device according to claim 18, wherein the first watch strap body is a non-elastic woven strap.

20. The wearable device according to claim 18 or 19, wherein one end of the first watch strap body comprises a third connection structure, the third connection structure comprises a first lug and a first end link, the air flow channel is provided in the first end link, and one end of the first end link is connected to the first end of the first watch strap body, and the other end of the first end link is connected to the side wall of the device body; and
the first lug is disposed to cover the first end link.

21. The wearable device according to claim 20, wherein the third connection structure further comprises a first buffer body, the first buffer body is disposed on the first end link, and the first buffer body is located between the first lug and the side wall of the device body.

22. The wearable device according to claim 20 or 21, wherein the third connection structure further comprises a cover plate and a fastener, the cover plate is pressed on the first air nozzle of the airbag, and the fastener is configured to fasten the cover plate to the first end link, to enable the airbag to be fastened to the first end link.

23. The wearable device according to any one of claims 18 to 22, further comprising: a fourth connection structure, wherein the fourth connection structure comprises a second lug, a second end link, and a spring bar, the second end link is connected to the second lug, and the second end link is connected to the device body; and
two ends of the spring bar are connected to the second lug, sliding space for being passed through by the second end of the first watch strap body is provided between the spring bar and the second lug, and the second end of the first watch strap body is slidably disposed around the spring bar.

24. The wearable device according to claim 23, wherein a bonding component is disposed on the second end of the first watch strap body, and a connecting component that is bonded to the bonding component is disposed on a side that is of the first watch strap body and that is disposed back to back the airbag.

25. The wearable device according to claim 23, wherein the fourth connection structure further comprises a second buffer body, the second buffer body is disposed on the second lug, and the second buffer body is located between the second lug and the side wall of the device body.

26. The wearable device according to any one of claims 18 to 25, wherein the airbag and the first watch strap body are connected by using a hook and loop fastener.

27. The wearable device according to any one of claims 1 to 26, wherein the pressure measurement assembly comprises a pressure difference sensor, an air path, and an air pump, and the vent is separately in communication with the pressure difference sensor and the air pump through the air path; and
a control valve is disposed in the air path.

28. A wearable system, comprising a terminal device and at least one wearable device according to any one of claims 1 to 27, wherein the terminal device is communicatively connected to the wearable device.
